(19) Europäisches Patentamt
European Patent Office
Office européen des brevets

(11) Numéro de publication: **0 076 718**
**B1**

(12) # FASCICULE DE BREVET EUROPEEN

(45) Date de publication du fascicule du brevet: **12.09.84**

(51) Int. Cl.³: **C 07 C 25/02**, C 07 C 17/12

(21) Numéro de dépôt: **82401695.0**

(22) Date de dépôt: **20.09.82**

(54) **Procédé de chloration de métadihalogénobenzènes.**

(30) Priorité: **02.10.81 FR 8118585**

(43) Date de publication de la demande: **13.04.83 Bulletin 83/15**

(45) Mention de la délivrance du brevet: **12.09.84 Bulletin 84/37**

(84) Etats contractants désignés: **AT BE CH DE FR GB IT LI LU NL SE**

(56) Documents cités:
**US - A - 4 186 153**
**US - A - 4 205 014**

(73) Titulaire: **RHONE-POULENC SPECIALITES CHIMIQUES, "Les Miroirs" 18, Avenue d'Alsace, F-92400 Courbevoie (FR)**

(72) Inventeur: **Soula, Gérard, 33, rue Nungesser, F-69330 Meyzieu (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de chloration de métadihalogénobenzènes.

On connaît, dans l'art antérieur, des procédés de chloration de composés halogénés qui font intervenir une catalyse acide.

C'est ainsi que la chloration à l'aide de chlore gazeux de tels substrats est largement décrite en présence de chlorure ferrique ou de chlorure d'aluminium. On peut citer, notamment, l'ouvrage de C.A. Thomas «Anhydrous Aluminium Chloride in Organic Chemistry» (Reinhold Publishing Corporation).

La présence d'halogène sur le noyau aromatique oriente, dans les conditions précitées, la chloration en ortho-para. Expérimentalement, on observe une chloration privilégiée en para.

Sur les dérivés métadihalogénés, on observe essentiellement une chloration en position 4. Par exemple, le métadichlorobenzène donne principalement le trichloro-1,2,4 benzène.

La chloration sélective en position 2 ne peut être réalisée par cette voie. Ces composés sont obtenus dans l'art antérieur par des voies non directes.

La titulaire a découvert une voie directe de chloration en position 2 de ces substrats.

L'invention consiste en une mise en œuvre totalement différente de la réaction de chloration classique, puisqu'elle substitue à la catalyse acide de l'art antérieur une catalyse basique.

L'invention concerne donc un procédé de chloration de métadihalogénobenzènes en position ortho des deux atomes d'halogène, caractérisé en ce que l'on fait réagir le métadihalogénobenzène non substitué en cette position avec un composé donneur de chlore en présence d'au moins un amidure alcalin et d'au moins un composé complexant le cation de l'amidure, le complexe formé étant soluble dans le milieu réactionnel et permettant la réaction avec le composé donneur de chlore.

Selon un premier mode de réalisation particulier du procédé selon l'invention, le composé complexant le cation de l'amidure est un agent séquestrant de formule générale (I):

$N[-CHR_1-CHR_2-O-(CHR_3-CHR_4-O)_n-R_5]_3$

dans laquelle n est un nombre entier supérieur ou égal à 0 et inférieur ou égal à 10 ($0 \leqslant n \leqslant 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $-C_mH_{2m}-\phi$, ou $C_mH_{2m+1}-\phi-$, m étant compris entre 1 et environ 12.

Selon un deuxième mode de réalisation particulier du procédé selon l'invention, le composé complexant le cation de l'amidure est un polyéther macrocyclique ayant de 15 à 30 atomes dans le cycle et constitué de 4 à 10 unités $-O-X$ dans lesquelles X est soit $-CHR_6-CHR_7-$ soit $-CHR_6-CHR_8-CR_9R_7-$, $R_6$, $R_7$, $R_8$ et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités $-O-X$ comprennent le groupement $-O-CHR_6-CHR_7-$.

Selon un troisième mode de réalisation particulier de l'invention, le composé complexant le cation de l'amidure est un composé macrocyclique ou bicyclique de formule générale:

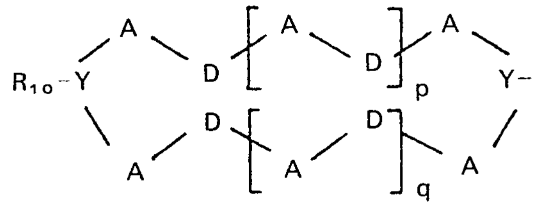
IIa

ou

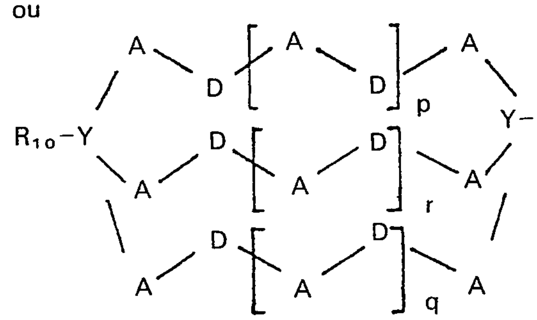
IIb

dans lesquelles:

– Y représente N ou P

– A représente un groupement alkylène ayant de 1 à 3 atomes de carbone.

– D représente O, S ou $N-R_{11}$ où $R_{11}$ représente un radical alkyle ayant de 1 à 6 atomes de carbone.

– $R_{10}$ représente un radical alkyle ayant de 1 à 6 atomes de carbone, p, q et r identiques ou différents sont des nombres entiers compris entre 1 et 5.

On ne sort pas du cadre de la présente invention en mettant en œuvre au moins deux des composés complexants définis ci-dessus.

L'amidure alcalin mis en œuvre dans le cadre de la présente invention est, de préférence, choisi parmi le groupe comprenant l'amidure de sodium, l'amidure de potassium et l'amidure de lithium.

L'amidure de sodium est particulièrement préféré.

Le composé donneur de chlore est de préférence choisi parmi le groupe comprenant l'hexachlorobenzène et le pentachlorobenzène.

Les métadihalogénobenzènes concernés par la présente invention peuvent être représentés par la formule générale:

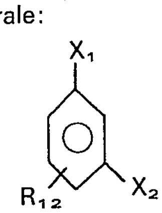
(III)

dans laquelle $X_1$ et $X_2$ identiques ou différents représentent chacun un élément choisi parmi le groupe comprenant Cl, F, et dans laquelle $R_{12}$ ne pouvant être en position 2 représente un radical choisi parmi le groupe comprenant les radicaux Cl,

F, les radicaux alkyle ayant de 1 à 6 atomes de carbone.

On peut citer comme exemples de tels composés, les produits suivants: le métadichlorobenzène, le métadifluorobenzène, le chlorodifluoro-2,4 benzène et le difluoro-2,4 toluène.

On peut représenter les produits obtenus par la formule générale:

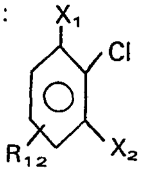

(IV)

où $X_1$, $X_2$ et $R_{12}$ ont la signification précédente.

Ces composés sont d'utiles intermédiaires de synthèse.

L'invention est particulièrement intéressante pour la préparation des composés suivants: le difluoro-1,3 chloro-2 benzène, le difluoro-2,4 chloro-3 toluène, le dichloro-2,3 fluorobenzène et le dichloro-1,3 difluoro-2,4 benzène.

Selon un mode de réalisation préférentiel du procédé de l'invention, on utilise au moins un agent séquestrant de formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en œuvre les agents séquestrants pour lesquels n est supérieur ou égal à 0 et inférieur ou égal à 6, et pour lesquels $R_5$ représente un radical alkyle ayant de 1 à 4 atomes de carbone.

On peut citer:

– la tris(oxa-3 butyl)amine de formule:
$N(-CH_2-CH_2-O-CH_3)_3$

– la tris(oxa-3 heptyl)amine de formule:
$N(-CH_2-CH_2-O-C_4H_9)_3$

– la tris(dioxa-3,6 heptyl)amine de formule: $N(-CH_2-CH_2-O-CH_2-CH_2-O-CH_3)_3$

– la tris(trioxa-3,6,9 décyl)amine de formule:
$N(-CH_2-CH_2-O-CH_2-CH_2-O-CH_3)_3$

– la tris(dioxa-3,6 octyl)amine de formule:
$N(-CH_2-CH_2-O-CH_2-CH_2-O-C_2H_5)_3$

– la tris(trioxa-3,6,9 undécyl)amine de formule: $N(-CH_2-CH_2-O-CH_2-CH_2-O-CH_2-CH_2-O-C_2H_5)_3$

– la tris(dioxa-3,6 nonyl)amine de formule:
$N(-CH_2-CH_2-O-CH_2-CH_2-O-C_3H_7)_3$

– la tris(trioxa-3,6,9 dodécyl)amine de formule: $N(-CH_2-CH_2-O-CH_2-CH_2-O-CH_2-CH_2-O-C_3H_7)_3$

– la tris(dioxa-3,6 décyl)amine de formule:
$N(-CH_2-CH_2-O-CH_2-CH_2-O-C_4H_9)_3$

– la tris(trioxa-3,6,9 tridécyl)amine de formule: $N(-CH_2-CH_2-O-CH_2-CH_2-O-CH_2-CH_2-O-C_4H_9)_3$

– la tris(tétraoxa-3,6,9,12 tridécyl)amine de formule:
$N(-CH_2-CH_2-O-(-CH_2-CH_2-O-)_3-CH_3)_3$

– la tris(dioxa-3,6 méthyl-4 heptyl)amine de formule:
$N(-CH_2-CH_2-O-CHCH_3-CH_2-O-CH_3)_3$

– la tris(dioxa-3,6 diméthyl-2,4 heptyl)amine de formule:
$N-(CH_2-CHCH_3-O-CHCH_3-CH_2-O-CH_3)_3$

La préparation de ces agents séquestrants est décrite dans la demande de brevet français 79.05438 publiée sous le N° 2450120.

Les polyéthers macrocycliques qui peuvent être mis en œuvre dans le procédé selon l'invention sont connus sous l'appellation générale d'«éthers couronnes» et sont décrits dans le brevet français 69.43879 publié sous le N° 2026481.

On peut citer comme exemples d'éthers couronnes pouvant être utilisés selon l'invention:

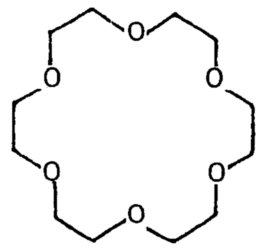

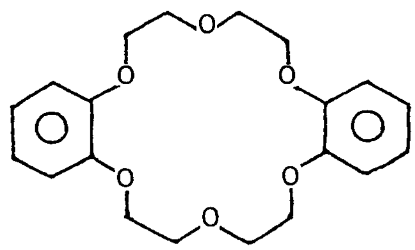

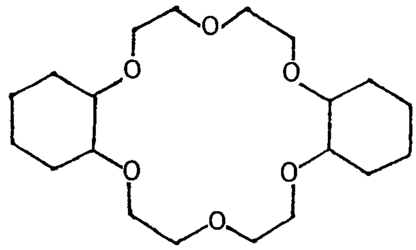

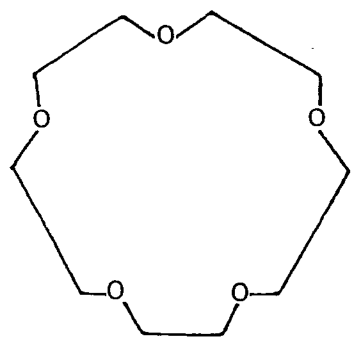

Les composés macrocycliques et bicycliques sont décrits dans le brevet français 70.21079 publié sous le N° 2052947. On peut citer comme exemples de tels composés convenant pour la mise en œuvre du procédé selon l'invention:

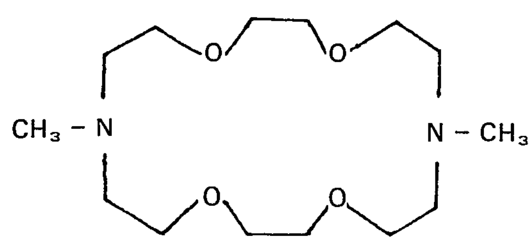

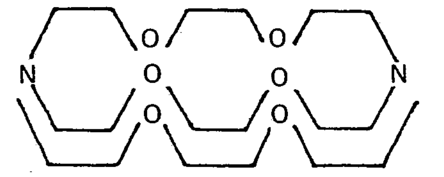

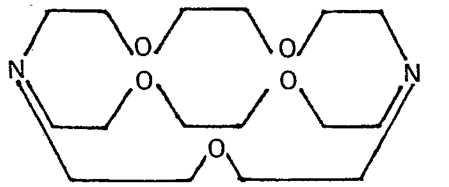

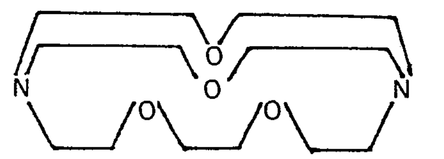

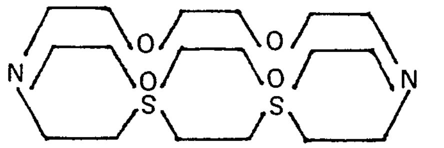

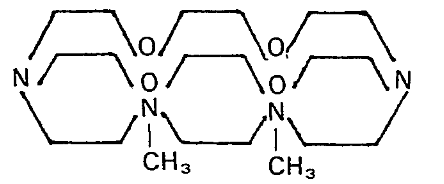

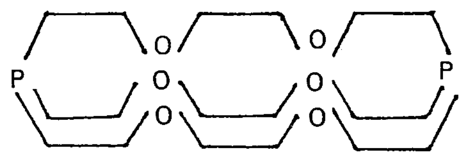

Le procédé selon l'invention peut être mis en œuvre en présence ou non d'un tiers solvant. Quand on en utilise un, on le choisit de préférence parmi les solvants aprotiques apolaires ou aprotiques peu polaires comme par exemple le benzène, le toluène, le chlorobenzène.

On peut ne pas utiliser de tiers solvant; c'est alors l'agent de chloration qui joue le rôle de solvant.

Selon l'interprétation de la titulaire, l'invention repose sur le fait que le produit de la réaction de l'amidure alcalin avec le métadihalogénobenzène, que l'on peut représenter par la formule suivante, dans le cas où l'amidure alcalin est l'amidure de sodium, est solubilisé pour tout ou partie dans le milieu réactionnel par complexation du cation par le composé complexant:

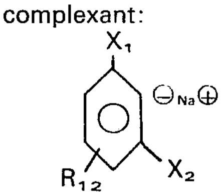

Cette solubilisation permet la réaction avec le donneur de chlore, ce dernier se transforme en son homologue inférieur (par exemple le pentachlorobenzène quand l'hexachlorobenzène est mis en œuvre).

Selon le procédé de l'invention, on utilise une quantité d'amidure alcalin telle que le rapport molaire de l'amidure alcalin au composé de formule III est compris de préférence entre 0,05 et 1. Encore plus préférentiellement, ce rapport est compris entre 0,1 et 0,5.

Le composé donneur de chlore est utilisé en quantité telle que le rapport molaire de ce composé au composé de formule III est de préférence compris entre 0,3 et 1.

Encore plus préférentiellement, ce rapport est compris entre 0,7 et 1.

Le composé complexant est utilisé en quantité telle que le rapport molaire de l'agent complexant au composé III est compris de préférence entre 0,01 et 0,2. Encore plus préférentiellement, ce rapport est compris entre 0,03 et 0,1.

La réaction est effectuée généralement à une température comprise entre environ −30°C et environ 50°C.

On préfère opérer à température ambiante sous pression atmosphérique.

L'invention va être maintenant plus complètement illustrée à l'aide des exemples qui vont suivre.

*Exemple 1:*

*Chloration du dichloro-1,3 benzène par l'hexachlorobenzène*

Dans un réacteur de 10 $cm^3$, muni d'une agitation magnétique, on charge successivement 1,5 g (0,01 mol) de dichloro-1,3 benzène dissous dans 1,5 g de chlorobenzène et 1,42 g (0,005 mol) d'hexachlorobenzène.

On ajoute alors 0,16 g de tris(dioxa-3,6 heptyl)-amine soit 5% en moles par rapport au dichloro-1,3 benzène.

La température du mélange est maintenue sous agitation à 20°C, on introduit 0,15 g d'amidure de sodium $NaNH_2$ en solution 50% dans du toluène (0,002 mol).

Après une heure de contact, on analyse le mélange brut réactionnel par chromatographie en phase vapeur.

La composition du mélange est la suivante: trichloro-1,2,3 benzène (90%), trichloro-1,2,4 benzène (5%), trichloro-1,3,5 benzène (5%). Le taux de transformation est de 62% et le rendement 88%.

*Exemple 2:*

*Chloration du difluoro-1,3 benzène par l'hexachlorobenzène*

Dans un réacteur de 5 $cm^3$ muni d'une agitation magnétique, on charge successivement 0,57 g (0,005 mol) de difluoro-1,3 benzène dissous dans 0,57 g de chlorobenzène et 0,71 g (0,0025 mol) d'hexachlorobenzène.

On ajoute alors 0,08 gramme de tris(dioxa-3,6 heptyl)amine soit 5% en moles par rapport au difluoro-1,3 benzène.

La température du mélange est maintenue sous agitation à 20°C. On introduit 0,075 g d'amidure de sodium $NaNH_2$ en solution 50% dans du toluène (0,001 mol).

Après 30 minutes de contact, on analyse le mélange brut réactionnel par chromatographie en phase vapeur.

Le taux de transformation est de 50% environ et le rendement en 2,6 difluorochlorobenzène 87%.

*Exemple 3:*

*Chloration du fluoro-1 chloro-3 benzène par l'hexachlorobenzène*

Dans un réacteur de 10 $cm^3$ muni d'une agitation magnétique, on charge successivement 1,3 g (0,01 mol) de fluoro-1 chloro-3 benzène dissous dans 1,3 g de chlorobenzène et 1,42 g (0,005 mol) d'hexachlorobenzène.

On ajoute alors 0,16 g de tris(dioxa 3,6 heptyl)-amine soit 5% en moles par rapport au fluoro-1 chloro-3 benzène.

La température du mélange est maintenue sous agitation à 20°C. On introduit 0,14 g d'amidure de sodium $NaNH_2$ en solution 50% dans du toluène (0,0018 mol).

Après 20 minutes de contact, on analyse le brut réactionnel par chromatographie en phase vapeur.

Le dichloro-2,3 fluorobenzène est formé avec un rendement de 65%.

*Exemple 4:*

*Chloration du difluoro-2,4 chlorobenzène par l'hexachlorobenzène*

Dans un réacteur de 150 $cm^3$, muni d'une agitation magnétique, on charge successivement 22,2 g (0,15 mol) de difluoro-2,4 chlorobenzène dissous dans 22,2 g de toluène et 28,5 g (0,1 mol) d'hexachlorobenzène (28,5 g).

On ajoute alors 4,85 g de tris(dioxa-3,6 heptyl)-amine soit 5% en moles par rapport au difluoro-2,4 chlorobenzène.

La température du mélange est maintenue sous agitation à 20°C; on introduit 5,8 grammes d'amidure de sodium $NaNH_2$ en solution 50% dans du toluène (0,075 mol).

Après 20 minutes de contact, on analyse le brut réactionnel par chromatographie en phase vapeur.

Le difluoro-2,4 dichloro-1,3 benzène est obtenu avec un rendement de 61%.

**Revendications**

1. Procédé de chloration de métadihalogénobenzènes en position ortho des deux atomes d'halogène, caractérisé en ce que l'on fait réagir le métadihalogénobenzène non substitué en cette position avec un composé donneur de chlore en présence d'au moins un amidure alcalin et d'au moins un composé complexant le cation de l'amidure, le complexe formé étant soluble dans le milieu réactionnel et permettant la réaction avec le composé donneur de chlore.

2. Procédé selon la revendication 1 caractérisé en ce que selon l'invention, le composé complexant le cation de l'amidure est un agent séquestrant de formule générale (I):

$$N[-CHR_1-CHR_2-O-(CHR_3-CHR_4-O)_n-R_5]_3$$

dans laquelle n est un nombre entier supérieur ou égal à 0 et inférieur ou égal à 10 ($0 \leqslant n \leqslant 10$), $R_1$, $R_2$, $R_3$, $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule $-C_mH_{2m}-\phi$, ou $C_mH_{2m+1}-\phi-$, m étant compris entre 1 et environ 12.

3. Procédé selon la revendication 2, caractérisé en ce que, dans la formule (I) $R_1$, $R_2$, $R_3$, et $R_4$ représente un atome d'hydrogène ou un radical méthyle.

4. Procédé selon la revendication 2 ou 3, caractérisé en ce que dans la formule (I) n est un nombre entier supérieur ou égal à 0 et inférieur ou égal à 6.

5. Procédé selon l'une quelconque des revendications 2 à 4, caractérisé en ce que dans la formule (I) $R_5$ représente un radical alkyle ayant de 1 à 4 atomes de carbone.

6. Procédé selon les revendications 3, 4 et 5, caractérisé en ce que l'agent séquestrant de formule I est la tris(dioxa-3,6 heptyl)amine.

7. Procédé selon la revendication 1, caractérisé en ce que le composé complexant le cation de l'amidure est un polyéther macrocyclique ayant de 15 à 30 atomes dans le cycle et constitué de 4 à 10 unités $-O-X$ dans lesquelles X est soit $-CHR_6-CHR_7-$ soit $-CHR_6-CHR_8-CR_9R_7-$, $R_6$, $R_7$, $R_8$ et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités $-O-X$ comprennent le groupement $-O-CHR_6-CHR_7-$.

8. Procédé selon la revendication 7, caractérisé en ce que le polyéther macrocyclique est choisi parmi le groupe comprenant:

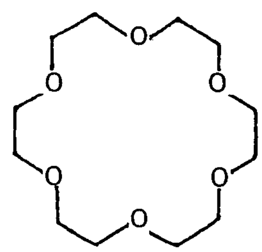

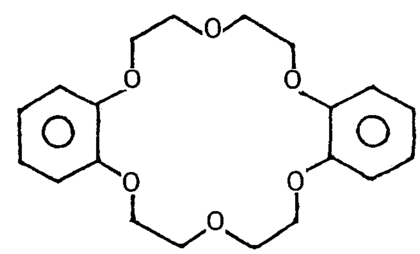

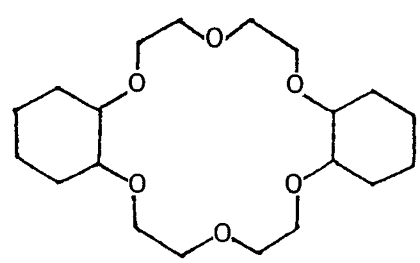

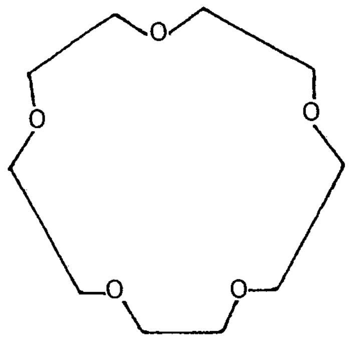

9. Procédé selon la revendication 1, caractérisé en ce que le composé complexant est un composé macrocyclique ou bicyclique de formule générale IIa ou IIb.

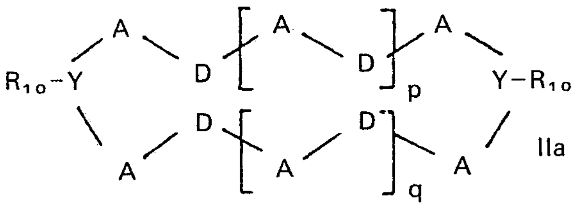

IIa

ou

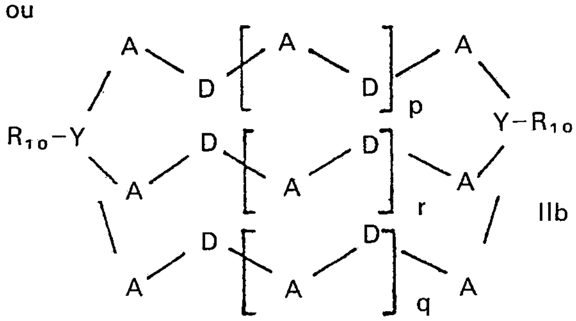

IIb

dans lesquelles:

– Y représente N ou P

– A représente un groupement alkylène ayant de 1 à 3 atomes de carbone

– D représente O, S ou $N-R_{11}$ où $R_{11}$ représente un radical alkyle ayant de 1 à 6 atomes de carbone

– $R_{10}$ représente un radical alkyle ayant de 1 à 6 atomes de carbone, p, q et r identiques ou différents sont des nombres entiers compris entre 1 et 5.

10. Procédé selon la revendication 9, caractérisé en ce que le composé macrocyclique ou bicyclique est choisi parmi le groupe comprenant:

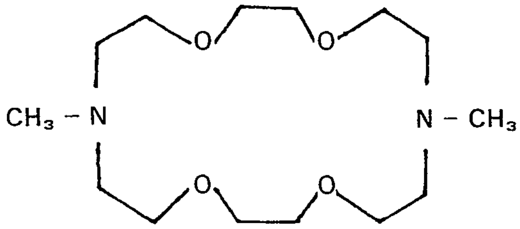

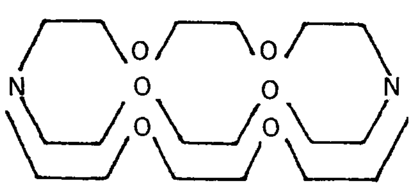

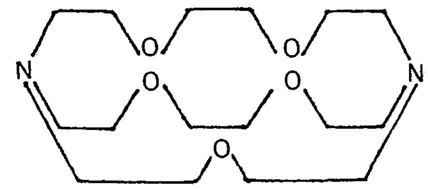

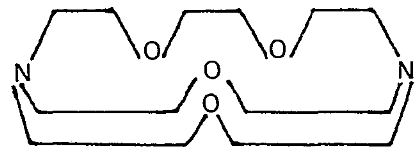

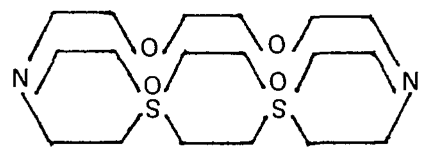

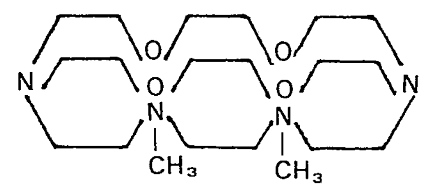

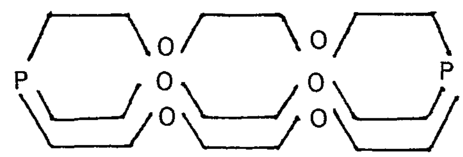

11. Procédé selon l'une quelconque des revendications précédentes, caractérisé en ce que l'on opère en présence d'un solvant aprotique apolaire ou aprotique peu polaire.

12. Procédé selon la revendication 11, caractérisà en ce que le solvant est choisi parmi le groupe comprenant le chlorobenzène, le benzène et le toluène.

13. Procédé selon l'une quelconque des revendications précédentes, caractérisé en ce que l'amidure alcalin est l'amidure de sodium ou l'amidure de potassium.

14. Procédé selon l'une quelconque des revendications précédentes, caractérisé en ce que le composé donneur de chlore est l'hexachlorobenzène ou le pentachlorobenzène.

15. Procédé selon l'une quelconque des revendications précédentes, caractérisé en ce que l'on utilise une quantité d'amidure alcalin telle que le rapport molaire de l'amidure alcalin au composé de formule III est compris entre 0,05 et 1.

16. Procédé selon l'une quelconque des revendications précédentes, caractérisé en ce que le composé donneur de chlore est utilisé en quantité telle que le rapport molaire de ce composé au composé de formule III est compris entre 0,3 et 1.

17. Procédé selon l'une quelconque des revendications précédentes, caractérisé en ce que le composé complexant est utilisé en quantité telle que le rapport molaire de l'agent complexant au composé III est compris de préférence entre 0,01 et 0,2.

18. Procédé selon l'une quelconque des revendications précédentes, caractérisé en ce que l'on opère à une température comprise entre −30°C et 50°C .

## Patentansprüche

1. Verfahren zur Chlorierung von Metadihalogenbenzolen in Ortho-Stellung der beiden Halogenatome, dadurch gekennzeichnet, dass man das in dieser Stellung unsubstituierte Metadihalogenbenzol mit einer Chlor liefernden Verbindung in Anwesenheit mindestens eines Alkaliamids und mindestens einer, das Kation des Amids komplexierenden Verbindung umsetzt, wobei der gebildete Komplex im Reaktionsgemisch löslich ist und mit der das Chlor liefernden Verbindung reagieren kann.

2. Verfahren nach Anspruch 1, dadurch gekennzeichnet, dass die das Kation des Amids komplexierende Verbindung ein Sequestrierungsmittel der allgemeinen Formel (I):
$N[-CHR_1-CHR_2-O-(CHR_3-CHR_4-O)_n-R_5]_3$
ist, in der n eine ganze Zahl gleich oder grösser 0 und kleiner als 10 ($0 \leqslant n \leqslant 10$) ist, $R_1$, $R_2$, $R_3$, $R_4$, die identisch oder verschieden sind, ein Wasserstoffatom oder einen Alkylrest mit 1 bis 4 Kohlenstoffatomen bedeuten und $R_5$ ein Alkyl- oder Cycloalkylrest mit 1 bis 12 Kohlenstoffatomen, ein Phenylrest oder ein Rest der Formel $-C_mH_{2m}-\phi$ oder $C_mH_{2m+1}-\phi-$ ist, wobei m zwischen 1 und etwa 12 liegt.

3. Verfahren nach Anspruch 2, dadurch gekennzeichnet, dass in der Formel (I) $R_1$, $R_2$, $R_3$ und $R_4$ ein Wasserstoffatom oder einen Methylrest bedeuten.

4. Verfahren nach Anspruch 2 oder 3, dadurch gekennzeichnet, dass in der Formel (I) n eine ganze Zahl gleich oder grösser 0 und kleiner oder gleich 6 ist.

5. Verfahren nach einem der Ansprüche 2 bis 4, dadurch gekennzeichnet, dass in der Formel (I) $R_5$ ein Alkylrest mit 1 bis 4 Kohlenstoffatomen ist.

6. Verfahren nach einem der Ansprüche 3, 4 oder 5, dadurch gekennzeichnet, dass das Sequestrierungsmittel der Formel (I) das Tris-(3,6-dioxaheptyl)amin ist.

7. Verfahren nach Anspruch 1, dadurch gekennzeichnet, dass die das Kation des Amids komplexierende Verbindung ein makrocyclischer Polyäther mit 15 bis 30 Ringatomen ist und aus 4 bis 10 O−X−Einheiten besteht, in denen X entweder $-CHR_6-CHR_7$ oder $-CHR_6-CHR_8-CR_9R_7-$ ist, wobei $R_6$, $R_7$, $R_8$ und $R_9$, identisch oder unterschiedlich ein Wasserstoffatom oder einen Alkylrest mit 1 bis 4 Kohlenstoffatomen bedeuten, und einer der Reste X, $-CHR_6-CHR_8-CR_9R_7$ sein kann, wenn die O−X−Einheiten die Gruppierung $-O-CHR_6-CHR_7$ enthalten.

8. Verfahren nach Anspruch 7, dadurch gekennzeichnet, dass das makrocyclische Polyäther aus den folgenden Gruppen gewählt ist:

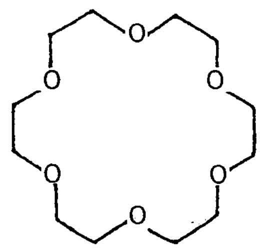

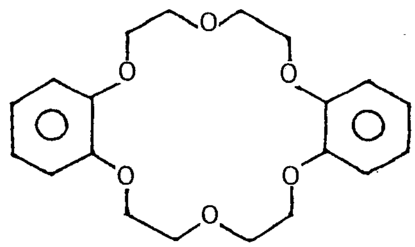

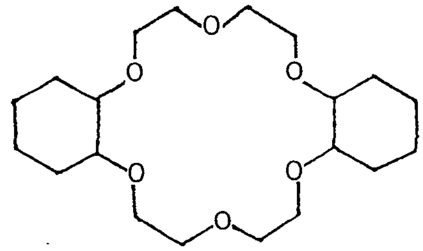

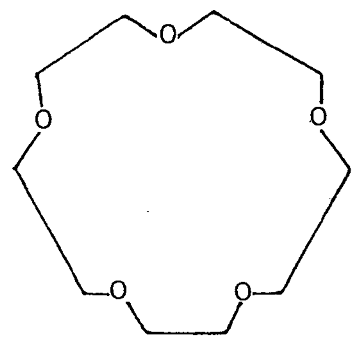

9. Verfahren nach Anspruch 1, dadurch gekennzeichnet, dass die den Komplex bildende Verbindung eine makro- oder bicyclische Verbindung von der allgemeinen Formel IIa oder IIb ist

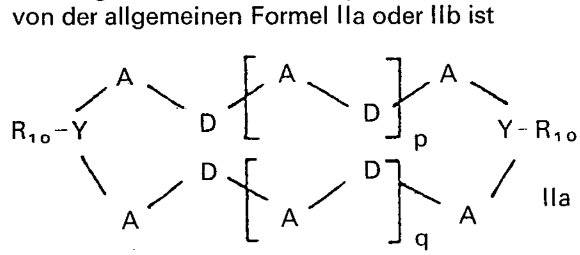

IIa

oder

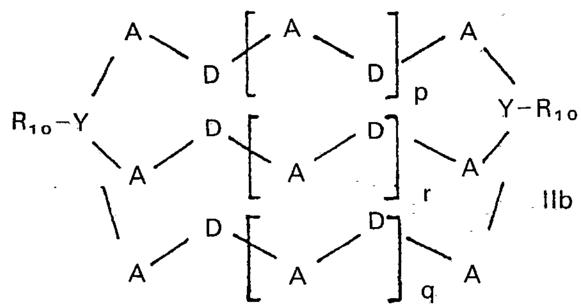

IIb

in denen:

– Y N oder P,

– A eine Alkylengruppe mit 1 bis 3 Kohlenstoffatomen,

– D O, S oder $N-R_{11}$ bedeutet, wobei $R_{11}$ ein Alkylrest mit 1 bis 6 Kohlenstoffatomen ist,

– $R_{10}$ einen Alkylrest mit 1 bis 6 Kohlenstoffatomen und p, q und r, die gleich oder verschieden sind, ganze Zahlen von 1 bis 5 bedeuten.

10. Verfahren nach Anspruch 9, dadurch gekennzeichnet, dass die makro- oder bicyclische Verbindung aus der folgenden Gruppe gewählt wird:

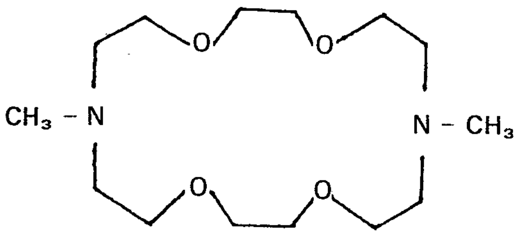

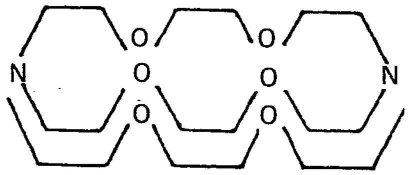

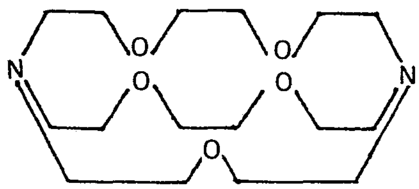

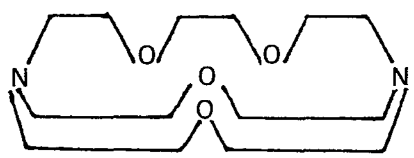

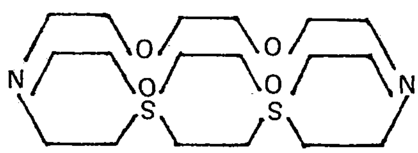

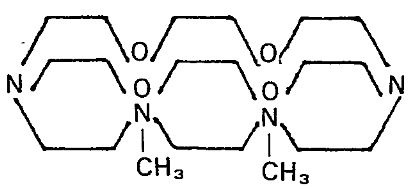

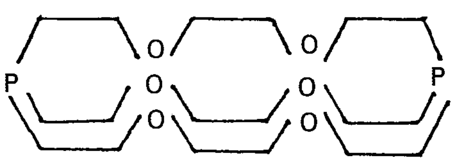

11. Verfahren nach irgendeinem der vorangehenden Ansprüche, dadurch gekennzeichnet, dass man in Anwesenheit eines apolaren aprotischen oder wenige polaren aprotischen Lösungsmittels arbeitet.

12. Verfahren nach Anspruch 11, dadurch gekennzeichnet, dass das Lösungsmittel aus der Gruppe von Chlorbenzol, Benzol und Toluol gewählt wird.

13. Verfahren nach irgendeinem der vorangehenden Ansprüche, dadurch gekennzeichnet, dass das Alkaliamid Natrium- oder Kaliumamid ist.

14. Verfahren nach irgendeinem der vorangehenden Ansprüche, dadurch gekennzeichnet, dass die Chlor liefernde Verbindung Hexachlorbenzol oder Pentachlorbenzol ist.

15. Verfahren nach irgendeinem der vorangehenden Ansprüche, dadurch gekennzeichnet, dass man eine solche Menge des Alkaliamids verwendet, dass das Molverhältnis des Alkaliamids zur Verbindung der Formel III zwischen 0,05 und 1 liegt.

16. Verfahren nach irgendeinem der vorangehenden Ansprüche, dadurch gekennzeichnet, dass die Chlor liefernde Verbindung in einer solche Menge verwendet wird, dass das Molverhältnis dieser Verbindung zur Verbindung der Formel III zwischen 0,3 und 1 liegt.

17. Verfahren nach irgendeinem der vorangehenden Ansprüche, dadurch gekennzeichnet, dass die komplexierende Verbindung in einer solchen Menge eingesetzt wird, dass das Molverhältnis des komplexierenden Mittels zur Verbindung III vorzugsweise 0,01 bis 0,2 beträgt.

18. Verfahren nach irgendeinem der vorangehenden Ansprüche, dadurch gekennzeichnet, dass man bei einer Temperatur zwischen −30°C und 50°C arbeitet.

## Claims

1. Process for the chlorination of meta-dihalobenzenes in an ortho-position to both halogen atoms, characterised in that the meta-dihalobenzene unsubstituted in this position is reacted with a chlorine-donating compound in the presence of at least one alkali metal amide and at least one compound complexing the cation of the amide, the complex formed being soluble in the reaction medium and permitting the reaction with the chlorine-donating compound.

2. Process according to Claim 1, characterised in that, according to the invention, the compound complexing the cation of the amide is a sequestering agent of the general formula (I):

$N[-CHR_1-CHR_2-O-(CHR_3-CHR_4-O)_n-R_5]_3$

in which n is an integer greater than or equal to 0 and smaller than or equal to 10 ($0 \leqslant n \leqslant 10$), $R_1$, $R_2$, $R_3$, $R_4$, which may be identical or different, denote a hydrogen atom or an alkyl radical containing from 1 to 4 carbon atoms and $R_5$ denotes an alkyl or cycloalkyl radical containing from 1 to 12 carbon atoms, a phenyl radical or a radical of the formula $-C_mH_{2m}-\phi$, or $C_mH_{2m+1}-\phi-$, m being between 1 and approximately 12.

3. Process according to Claim 2, characterised in that, in the formula (I) $R_1$, $R_2$, $R_3$, and $R_4$ denote a hydrogen atom or a methyl radical.

4. Process according to Claim 2 or 3, characterised in that in the formula (I) n is an integer

greater than or equal to 0 and smaller than or equal to 6.

5. Process according to any one of Claims 2 to 4, characterised in that in the formula (I) $R_5$ denotes an alkyl radical containing from 1 to 4 carbon atoms.

6. Process according to Claims 3, 4 and 5, characterised in that the sequestering agent of the formula I is tris(3,6-dioxaheptyl)amine.

7. Process according to Claim 1, characterised in that the compound complexing the cation of the amide is a macrocyclic polyether containing from 15 to 30 atoms in the ring and consisting of 4 to 10 $-O-X$ units in which X is either $-CHR_6-CHR_7-$ or $-CHR_6-CHR_8-CR_9R_7-$, $R_6$, $R_7$, $R_8$ and $R_9$, which may be identical or different, being a hydrogen atom or an alkyl radical containing from 1 to 4 carbon atoms, and one of the X may be $-CHR_6-CHR_8-CR_9R_7-$ when the $-O-X$ units include the group $-O-CHR_6-CHR_7-$.

8. Process according to Claim 7, characterised in that the macrocyclic polyether is chosen from the group comprising:

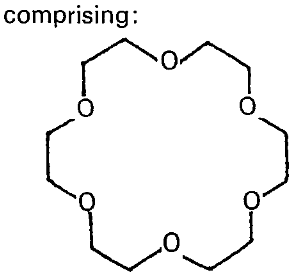

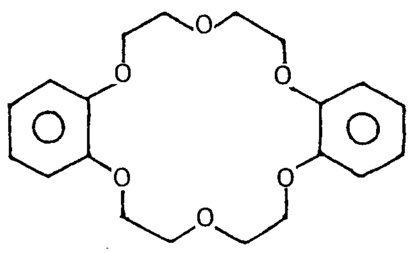

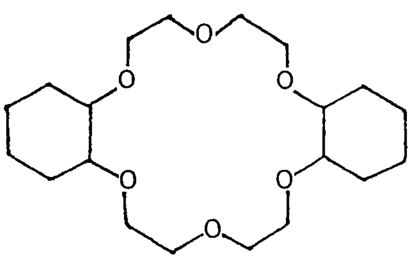

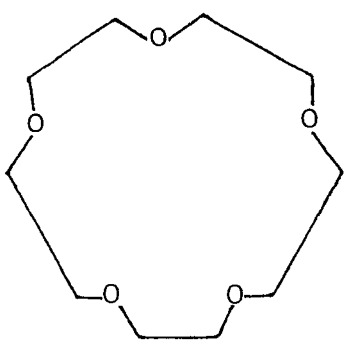

9. Process according to Claim 1, characterised in that the complexing compound is a macrocyclic or bicyclic compound of the general formula IIa or IIb.

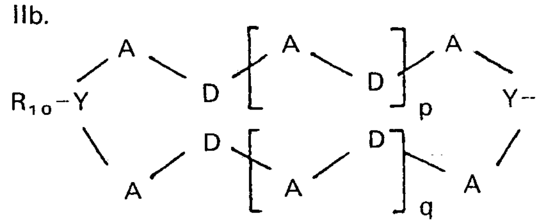

IIa

or

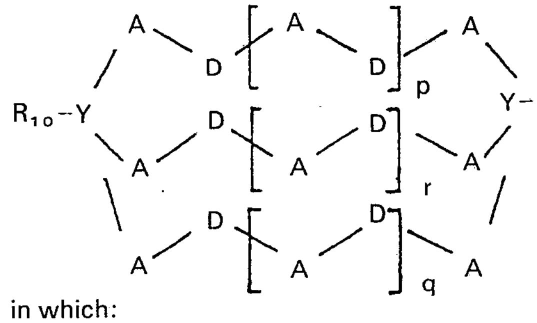

IIb

in which:

– Y denotes N or P

– A denotes an alkylene group containing from 1 to 3 carbon atoms

– D denotes O, S or $N-R_{11}$ where $R_{11}$ denotes an alkyl radical containing from 1 to 6 carbon atoms

– $R_{10}$ denotes an alkyl radical containing from 1 to 6 carbon atoms, and p, q and r, which may be identical or different, are integers between 1 and 5.

10. Process according to Claim 9, characterised in that the macrocyclic or bicyclic compound is chosen from the group comprising:

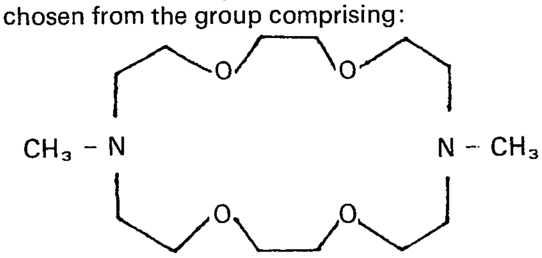

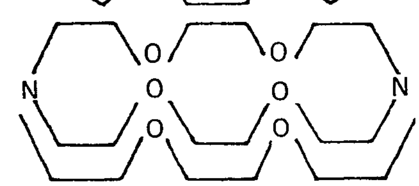

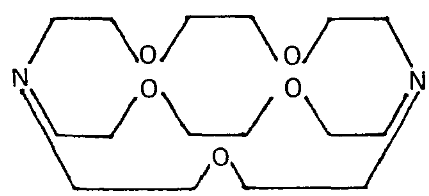

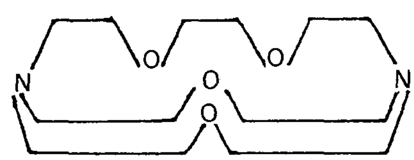

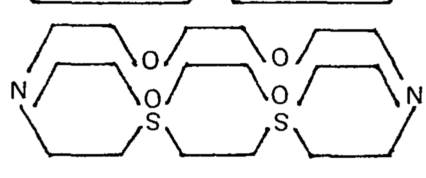

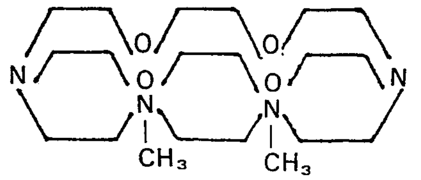

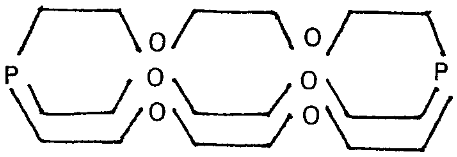

11. Process according to any one of the preceding claims, characterised in that it is carried out in the presence of an aprotic non-polar solvent or an aprotic weakly polar solvent.

12. Process according to Claim 11, characterised in that the solvent is chosen from the group comprising chlorobenzene, benzene and toluene.

13. Process according to any one of the preceding claims, characterised in that the alkali metal amide is sodium amide or potassium amide.

14. Process according to any one of the preceding claims, characterised in that the chlorine-donating compound is hexachlorobenzene or pentachlorobenzene.

15. Process according to any one of the preceding claims, characterised in that a quantity of alkali metal amide is employed such that the molar ratio of the alkali metal amide to the compound of formula III is between 0.05 and 1.

16. Process according to any one of the preceding claims, characterised in that the chlorine-donating compound is employed in a quantity such that the molar ratio of this compound to the compound of formula III is between 0.3 and 1.

17. Process according to any one of the preceding claims, characterised in that the complexing compound is employed in a quantity such that the molar ratio of the complexing agent to the compound III is preferably between 0.01 and 0.2.

18. Process according to any one of the preceding claims, characterised in that it is carried out at a temperature between −30°C and 50°C.